# EUROPÄISCHE PATENTANMELDUNG

(11) **EP 1 666 042 A2**
(43) Veröffentlichungstag der Anmeldung: **07.06.2006**
(21) Anmeldenummer: 06111485.6
(22) Anmeldetag: 07.04.2001
(51) Int. Cl.: A61K 31/55, A61K 31/4184, A61P 9/00

(54) **Arzneimittelkombination enthaltend Cilobradine und eine herzwirksame Verbindung, z.B. Telmisartan**

(30) Priorität: 13.04.2000 DE 10018401
(62) Teilanmeldung aus: 01949281.8
(71) Anmelder: Boehringer Ingelheim Pharma GmbH & Co. KG, 55216 Ingelheim am Rhein (DE)
(72) Erfinder: Die Erfindernennung liegt noch nicht vor

(57) **Zusammenfassung**

Die vorliegenden Erfindung betrifft die neue Verwendung von bradykarden Substanzen wie einen Ca⁺⁺ Kanal Blocker, Beta-Rezeptorenblocker oder i_{f}-Kanalblocker, wobei die i_{f}-Kanalblocker bevorzugt sind, gegebenenfalls in Kombination mit einer herzwirksamen Substanz zur Induktion der Regression von mit Hypertrophie einhergehenden Myocarderkrankungen, insbesondere zur Behandlung von idiopathischen hypertrophischen Kardiomyopathien (HCM) bei Mensch und Haustier.

## Beschreibung

Zur Behandlung einer erhöhten Herzfrequenz können bradykarde Substanzen eingesetzt werden, insbesondere Ca⁺⁺ Kanal Blocker wie Diltiazem und Verapamil oder Beta-Rezeptorenblocker wie Atenolol, Bisoprolol, Carvedolol, Metoprolol oder Propanolol und i_{f}-Kanalblocker wie Zatebradine [1-(7,8-Dimethoxy-1,3,4,5-tetrahydro-2H-3-benzazepin-2-on-3-yl)-3-[N-methyl-N-(2-(3,4-dimethoxy-phenyl)-ethyl)-propan] (siehe EP-B-0 065 229), 3-[(N-(2-(3,4-Dimethoxyphenyl)-ethyl)-piperidin-3-yl)-methyl]-(7,8-dimethoxy-1,3,4,5-tetrahydro-2H-3-benzazepin-2-on (siehe EP-B-0 224 794) und dessen Enantiomer Cilobradine [(+)-3-[(N-(2-(3,4-Dimethoxy-phenyl)-ethyl)-piperidin-3-(S)-yl)-methyl]-(7,8-dimethoxy-1,3,4,5-tetrahydro-2H-3-benzazepin-2-on] oder Alinidine [2-(N-Allyl-2,6-dichlor-anilino)-2-imidazolidin), siehe auch US-Patent Nr. 3,708,485], wobei von Zatebradine auch bekannt ist, dass es eine günstige Wirkung bei der Behandlung der Herzinsuffizienz (siehe EP-B-0 471 388) aufweist.

Ferner ist bekannt, dass bradykarde Substanzen, insbesondere die vorstehend erwähnten Verbindungen, wobei die i_{f}-Kanalblocker wie Zatebradine, Cilobradine oder Alinidine insbesondere jedoch Cilobradine bevorzugt sind, die Symptomatik von mit Hypertrophie einhergehenden Myocarderkrankungen, insbesondere zur Behandlung von idiopathischen hypertrophischen Kardiomyopathien (HCM) wie Hypertrophie des Restmyokards nach Herzinfarkt, ischämische Kardiomyopathie, Hypertrophie des Myokards bei Klappenvitien und Myokarditis bei toxischen oder iatrogenen Einflüssen positiv beeinflussen können.

Überraschenderweise wurde nun gefunden, dass bradykarde Substanzen, wobei die i_{f}-Kanalblocker wie Zatebradine, Cilobradine oder Alinidine insbesondere jedoch Cilobradine bevorzugt sind, nicht nur die klinische Symptomatik einer hypertrophischen Cardiomyopathie günstig beeinflussen, sondern sogar eine Regression dieser schweren Herzerkrankungen, induzieren.

Gegenstand der vorliegenden Erfindung ist somit die neue Verwendung von bradykarden Substanzen, insbesondere die der vorstehend erwähnten Verbindungen, wobei die i_{f}-Kanalblocker wie Zatebradine, Cilobradine oder Alinidine insbesondere jedoch Cilobradine bevorzugt sind, zur Induktion der Regression von mit Hypertrophie einhergehenden Myocarderkrankungen, insbesondere zur Behandlung von idiopathischen hypertrophischen Kardiomyopathien (HCM) bei Mensch und Haustier.

Zur Erzielung der erfindungsgemäßen Wirkung wird zweckmäßigerweise die zur Behandlung der erhöhten Herzfrequenz aus der Literatur für die einzelnen bradykarden Substanzen bekannte Dosierung verwendet. Beispielsweise beträgt die Einzeldosis
für Cilobradine 0.1 bis 0.5 mg/kg per os, vorzugsweise 0.2 bis 0.4 mg/kg, 1 bis 3 x täglich,
für Zatebradine 0.2 bis 1 mg/kg 2 x täglich sowie
für Alinidine 0.5 bis 5 mg/kg 2 x täglich.

Die erfindungsgemäße neue Verwendung der bradykarden Substanzen wurde am Beispiel des i_{f}-Kanalblockers Cilobradine wie folgt geprüft:
Eine Katze mit schwerer hypertrophischer Kardiomyopathie (Herzfrequenzen ca. 200 Schläge/Minuten), EKG mit ST-Anhebungen als Zeichen myokardialer Ischämie, erhöhter Kreatininkinaseaktivität im Plasma und im Ultraschallbild, massive Verdichtung der Ventrikelwand bei Reduktion des Ventrikelvolumens und der Auswurffraktion, zeigte nach Behandlung mit dem i_{f}-Kanalblocker Cilobradine (2 x täglich 0.3 mg/kg per os) eine deutliche Verbesserung der klinischen Symptomatik (Nachlassen der Schmerzhaftigkeit,
EKG-Normalisierung, Wiederaufnahme des normalen physiologischen Aktivitätsmusters).

Bei Nachuntersuchungen nach einem Jahr und nach ca. 2 Jahren Behandlungsdauer zeigt sich überraschenderweise eine Regression der Myokardhypertrophie bei weiterhin erhaltener Verbesserung der Symptomatik.

Die hypertrophische Kardiomyopathie der Katze gilt als Modell für die entsprechende Erkrankung der Menschen (Kittleson et al., Circulation **91,** 3172-3180 (1999)).

Die Behandlung mit dem i_{f}-Kanalblocker Cilobradine führt somit neben einer Verbesserung der Symptomatik zu einer Regression der Erkrankung.

Ein weiterer Gegenstand der vorliegenden Erfindung sind Arzneimittelkombinationen, enthaltend mindestens eine bradykarde Substanz, insbesondere eine der vorstehend erwähnten Verbindungen, wobei ein i_{f}-Kanalblocker bevorzugt ist, und mindestens eine herzwirksame Substanz wie
ein Herzglycosid, z.B. Methyldigoxin oder Digitoxin,
einen Vasodilatator, z.B. Nitroglycerin,
einen ACE-Hemmer, z.B. Captopril oder Enalapril,
einen Angiotensin-II-Antagonisten, z.B. Losartan oder Telmisartan,
welche ebenfalls zur Behandlung von mit Hypertrophie einhergehenden Myocarderkrankungen, insbesondere zur Behandlung von idiopathischen hypertrophischen Kardiomyopathien (HCM) geeignet sind, wenn durch eine Kombination mit einer bradykarden Substanz ein Anstieg der Herzfrequenz verhindert werden kann.

Zur Erzielung der erfindungsgemäßen Wirkung wird zweckmäßigerweise die zur Behandlung der erhöhten Herzfrequenz aus der Literatur für die einzelnen bradykarden Substanzen sowie die der für die eingesetzte herzwirksame Verbindung aus der Literatur bekannten Dosierungen verwendet.

Hierzu werden die bradykarden Substanzen entweder alleine oder in Kombination mit anderen herzwirksamen Verbindungen mit
einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragées, Kapseln, Pulver, Suspensionen, Lösungen, Sprays oder Zäpfchen eingearbeitet.

So enthält beispielsweise die Kombination bestehend aus Cilobradine und einer herzwirksamen Verbindung, zweckmäßigerweise 0.1 bis 0.5 mg/kg, vorzugsweise 0.2 bis 0.4 mg/kg Cilobradine per os
plus 0.01 bis 1 mg Methyldigoxin, 1 bis 2 x täglich,
0.01 bis 1 mg Digoxin, 1 x täglich,
0.1 bis 2 mg Nitroglycerin, 2 bis 3 x täglich,
10 bis 100 mg Captopril, 1 bis 2 x täglich,
2 bis 20 mg Enalapril, 1 x täglich,
10 bis 200 mg Losartan, 2 x täglich, oder
20 bis 80 mg Telmisartan, 1 x täglich.

Da die Kombinationspartner für die i_{f}-Kanalblocker zusätzlich an unabhängigen biologischen System angreifen und i_{f}-Kanalblocker reflektorische Herzfrequenzerhöungen hemmen, welche im Zusammenhang mit den obigen Kombinationspartner auftreten können, weisen diese eine synergistische Wirkungsweise auf.

Die nachfolgenden Beispiele sollen die Erfindung näher erläutern, ohne diese zu beschränken:

### Beispiel 1

### Kapseln zu 1,25 mg Cilobradine

Zusammensetzung:

| 1 Kapsel enthält: | | |
|---|---|---|
| | Lactosemonohydrat | 82,75 mg |
| | Maisstärke | 55,3 mg |

### Herstellungsverfahren

Der Wirkstoff, Lactosemonohydrat und Maisstärke werden gemischt und in Kapseln Größe 4 abgefüllt.

### Beispiel 2

### Kapseln zu 10 mg Cilobradine

Zusammensetzung:

| 1 Kapsel enthält: | | |
|---|---|---|
| | Lactosemonohydrat | 77,6 mg |
| | Maisstärke | 51,7 mg |

### Herstellungsverfahren

Der Wirkstoff, Lactosemonohydrat und Maisstärke werden gemischt und in Kapseln Größe 4 abgefüllt.

### Beispiel 3

### Tabletten zu 7,5 mg Cilobradine

Zusammensetzung:

| 1 Tablette enthält: | | |
|---|---|---|
| | Wirksubstanz | 7,5 mg |
| | Maisstärke | 59,5 mg |
| | Milchzucker | 48,0 mg |
| | Polyvinylpyrrolidon | 4,0 mg |
| | Magnesiumstearat | 1,0 mg |
| | | 120,0 mg |

### Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten. Tablettengewicht: 120 mg

### Beispiel 4

### Dragées zu 5 mg Cilobradine

| 1 Dragéekern enthält: | | |
|---|---|---|
| | Wirksubstanz | 5,0 mg |
| | Maisstärke | 41,5 mg |
| | Milchzucker | 30,0 mg |
| | Polyvinylpyrrolidon | 3,0 mg |
| | Magnesiumstearat | 0,5 mg |
| | | 80,0 mg |

### Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragees werden mit Wachs poliert.
Drageegewicht: 130 mg

### Beispiel 5

### Ampullen zu 5 mg Cilobradine

| 1 Ampulle enthält: | | |
|---|---|---|
| | Wirksubstanz | 5,0 mg |
| | Sorbit | 50,0 mg |
| | Wasser für Injektionszwecke ad | 2,0 mg |

### Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter N₂-Begasung in gereinigte und sterilisierte Ampullen abgefüllt und 20 Minuten im strömenden Wasserdampf autoklaviert.

### Beispiel 6

### Suppositorien zu 10 mg Cilobradine

| 1 Zäpfchen enthält: | | |
|---|---|---|
| | Wirksubstanz | 0,010 g |
| | Hartfett (z.B. Witepsol H 19 und W 45) | 1,690 g |
| | | 1,700 g |

### Herstellungsverfahren

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

### Beispiel 7

### Tropfenlösung mit 10 mg Cilobradine

| 100 ml Lösung enthalten: | | |
|---|---|---|
| | Wirksubstanz | 0,2 g |
| | Hydroxyethylcellulose | 0,15 g |
| | Weinsäure | 0,1 g |
| | Sorbitlösung 70 % Trockensubstanz | 30,0 g |
| | Glycerin | 10,0 g |
| | Benzoesäure | 0,15 g |
| | Dest. Wasser | ad 100 ml |

### Herstellungsverfahren

Dest. Wasser wird auf 70°C erhitzt. Hierin wird unter Rühren Hydroxyethylcellulose, Benzoesäure und Weinsäure gelöst. Es wird auf Raumtemperatur abgekühlt und hierbei das Glycerin und
die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Patentansprüche

1. Arzneimittelkombination enthaltend Cilobradine und eine herzwirksame Verbindung ausgewählt aus ein Herzglycosid, einen Vasodilatator, einen ACE-Hemmer oder einen Angiotensin-II-Antagonisten.

2. Arzneimittelkombination enthaltend Cilobradine und Telmisartan.
